# EUROPÄISCHE PATENTSCHRIFT

(11) **EP 0 546 387 B1**
(45) Veröffentlichungstag und Bekanntmachung des Hinweises auf die Patenterteilung: **31.01.1996**
(21) Anmeldenummer: 92120186.9
(22) Anmeldetag: 26.11.1992
(51) Int. Cl.: C07C 251/40, A01N 37/50, C07C 237/22

(54) **Substituierte Oximetheramide**
Substituted oximetheramides
Oximetheramides substitués

(30) Priorität: 09.12.1991 DE 4140558
(43) Veröffentlichungstag der Anmeldung: 16.06.1993
(73) Patentinhaber: BAYER AG, D-51368 Leverkusen (DE)
(72) Erfinder: Krämer, Wolfgang, Dr., W-5093 Burscheid 2 (DE); Berg, Dieter, Prof. Dr., W-5600 Wuppertal (DE); Dehne, Heinz-Wilhelm, Dr., W- 4019 Monheim (DE); Dutzmann, Stefan, Dr., W-4010 Hilden 1 (DE); Erdelen, Christoph, Dr., W-5653 Leichlingen 3 (DE)

(56) Entgegenhaltungen:
- EP-A- 0 464 381
- CHEMICAL ABSTRACTS, vol. 95, 1981, Columbus, Ohio, US; abstract no. 147465a,
- CHEMICAL ABSTRACTS, vol. 102, 1985, Columbus, Ohio, US; abstract no. 204258z,
- TETRAHEDRON LETTERS Bd. 23, Nr. 36, 1982, Seiten 3699 - 3702 S. NISHIYAMA ET AL

## Beschreibung

Die Erfindung betrifft neue substituierte Oximetheramide, ein Verfahren zu ihrer Herstellung und ihre Verwendung als Schädlingsbekämpfungsmittel.

Es ist bekannt, daß bestimmte substituierte Acrylester-Derivate wie beispielsweise die Verbindung 3-Methoxy-2-(2-methylphenyl)-acrylsäuremethylester oder die Verbindung 3-Methoxy-2-(2-phenoxyphenyl)-acrylsäuremethylester fungizide Eigenschaften besitzen (vergleiche z.B. EP 178 826).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Oximetheramide der allgemeinen Formel (I), 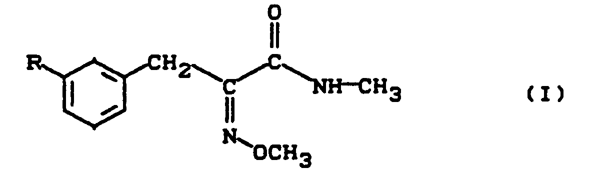
in welcher
- R: für gegebenenfalls substituiertes Aryl oder für einen Rest der Formel 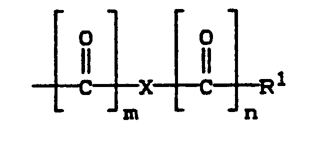 steht,
wobei
- R¹: für Alkyl, Halogenalkyl, Alkoxyalkyl, Alkoxycarbonylalkyl oder für jeweils gegebenenfalls substituiertes Cycloalkyl, Cycloalkylalkyl, Aryl, Arylalkyl, Heterocyclyl oder Heterocyclylalkyl steht,
- X: für Sauerstoff oder einen Rest der Formel N-R² steht,
- R²: für Wasserstoff oder Alkyl steht,
- m: für eine Zahl 0 oder 1 steht und
- n: für eine Zahl 0 oder 1 steht, gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Oximetheramide der allgemeinen Formel (I), 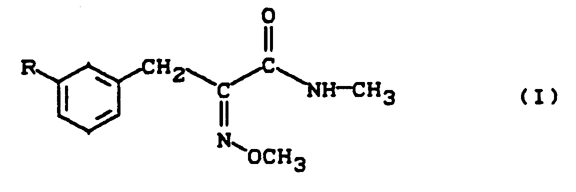
in welcher
- R: für gegebenenfalls substituiertes Aryl oder für einen Rest der Formel 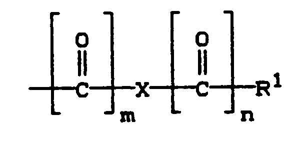 steht,
wobei
- R¹: für Alkyl, Halogenalkyl, Alkoxyalkyl, Alkoxycarbonylalkyl oder für jeweils gegebenenfalls substituiertes Cycloalkyl, Cycloalkylalkyl, Aryl, Arylalkyl, Heterocyclyl oder Heterocyclylalkyl steht,
- X: für Sauerstoff oder einen Rest der Formel N-R² steht,
- R²: für Wasserstoff oder Alkyl steht,
- m: für eine Zahl 0 oder 1 steht und
- n: für eine Zahl 0 oder 1 steht,
erhält, wenn man
a) Acrylamid-Derivate der Formel (II), 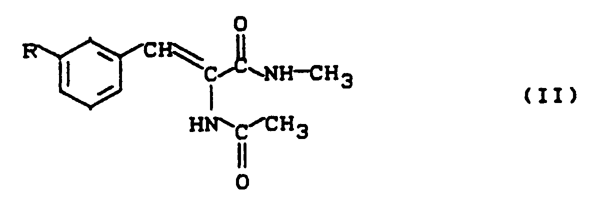 in welcher
   - R: die oben angegebene Bedeutung hat,
   mit einem Säureadditionssalz des O-Methylhydroxylamins gegebenenfalls in Gegenwart eines Verdünnungsmittels und gegebenenfalls in Gegenwart eines Reaktionshilfsmittels umsetzt oder wenn man
b) 3-(3-Hydroxyphenyl)-2-methoximino-propionsäure-N-methylamid der Formel (III), 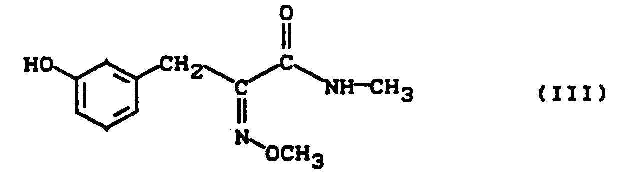
   mit Alkylierungs- oder Acylierungsmitteln der Formel (IV), 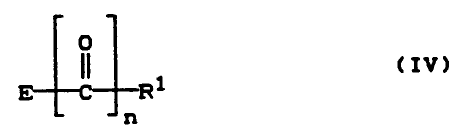 in welcher
   - R¹: und n die oben angegebene Bedeutung haben und
   - E: für eine elektronenanziehende Abgangsgruppe steht,
   gegebenenfalls in Gegenwart eines Verdünnungsmittels und gegebenenfalls in Gegenwart eines Reaktionshilfsmittels acyliert oder alkyliert.

Schließlich wurde gefunden, daß die neuen substituierten Oximetheramide der allgemeinen Formel (I) gute Wirksamkeit gegen Schädlinge besitzen.

Überraschenderweise zeigen die erfindungsgemäßen substituierten Oximetheramide der allgemeinen Formel (I) eine erheblich bessere Wirksamkeit gegen pflanzenschädigende Pilze und Insekten als die aus dem Stand der Technik bekannten substituierten Acrylester-Derivate, wie beispielsweise die Verbindung 3-Methoxy-2-(2-methylphenyl)-acrylsäuremethylester oder die Verbindung 3-Methoxy-2-(2-phenoxyphenyl)acrylsäuremethylester, welche chemisch und wirkungsmäßig naheliegende Verbindungen sind.

Die erfindungsgemäßen substituierten Oximetheramide sind durch die Formel (I) allgemein definiert. Bevorzugt sind Verbindungen der Formel (I), bei welchen
- R: für gegebenenfalls einfach oder mehrfach, gleich oder verschieden substituiertes Aryl mit 6 bis 10 Kohlenstoffatomen steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy oder Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 1 bis 11 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden durch Halogen und/oder geradkettiges oder verzweigtes Alkyl und/oder Alkoxy mit 1 bis 4 Kohlenstoffatomen substituiertes Phenyl, Phenoxy, Phenylthio, Phenylcarbonyl, Phenylcarbonyloxy, Phenylcarbonylamino, Phenylcarbonyl-(N-alkyl)-amino, Phenylaminocarbonyl, N-Phenyl-N-alkyl-aminocarbonyl, Phenylalkyl oder Phenylalkenyl mit jeweils gegebenenfalls bis zu 6 Kohlenstoffatomen in den einzelnen Alkyl- bzw. Alkenylteilen und
- R: außerdem für einen Rest der Formel 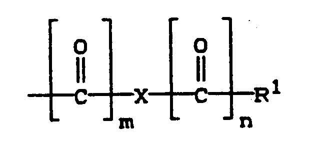 steht,
wobei
- R¹: für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxyalkyl oder Alkoxycarbonylalkyl mit jeweils 1 bis 6 Kohlenstoffatomen in den einzelnen Alkylteilen oder für jeweils gegebenenfalls einfach oder mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil steht, wobei als Cycloalkylsubstituenten jeweils infrage kommen: Halogen und/oder geradkettiges oder verzweigtes Alkyl mit 1 bis 4 Kohlenstoffatomen,
- R¹: außerdem für jeweils gegebenenfalls einfach oder mehrfach, gleich oder verschieden substituiertes Aryl oder Arylalkyl mit jeweils 6 bis 10 Kohlenstoffatomen im Arylteil und gegebenenfalls 1 bis 6 Kohlenstoffatomen im geradkettigen oder verzweigten Alkylteil steht oder für jeweils gegebenenfalls einfach oder mehrfach, gleich oder verschieden substituiertes, im Heterocyclylteil gesättigtes oder ungesättigtes Heterocyclyl oder Heterocyclylalkyl mit jeweils 2 bis 9 Kohlenstoffatomen und 1 bis 4 gleichen oder verschiedenen Heteroatomen - insbesondere Stickstoff, Sauerstoff und/oder Schwefel - im Heterocyclylteil und gegebenenfalls 1 bis 6 Kohlenstoffatomen im geradkettigen oder verzweigten Alkylteil steht, wobei als Aryl- oder Heterocyclylsubstituenten jeweils die bei dem Substituenten R genannten infrage kommen,
- X: für Sauerstoff oder einen Rest der Formel N-R² steht,
- R²: für Wasserstoff oder für geradkettiges oder verzweigtes Alkyl mit 1 bis 6 Kohlenstoffatomen steht,
- m: für eine Zahl 0 oder 1 steht und
- n: für eine Zahl 0 oder 1 steht.

Besonders bevorzugt sind Verbindungen der Formel (I), bei welchen
- R: für gegebenenfalls einfach bis fünffach, gleich oder verschieden substituiertes Aryl mit 6 oder 10 Kohlenstoffatomen steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 5 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 5 Kohlenstoffatomen und 1 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden durch Halogen und/oder jeweils geradkettiges oder verzweigtes Alkyl und/oder Alkoxy mit jeweils 1 bis 3 Kohlenstoffatomen substituiertes Phenyl, Phenoxy, Phenylthio, Phenylcarbonyl, Phenylcarbonyloxy, Phenylcarbonylamino, Phenylcarbonyl-(N-alkyl)-amino, Phenylaminocarbonyl, N-Phenyl-N-alkyl-aminocarbonyl, Phenylalkyl oder Phenylalkenyl mit jeweils gegebenenfalls bis zu 4 Kohlenstoffatomen in den einzelnen Alkyl- bzw. Alkenylteilen und
- R: außerdem für einen Rest der Formel 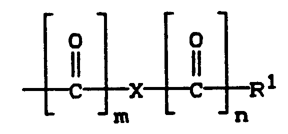 steht,
wobei
- R¹: für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkoxycarbonylalkyl mit jeweils 1 bis 4 Kohlenstoffatomen in den einzelnen Alkylteilen oder für jeweils gegebenenfalls einfach bis vierfach, gleich oder verschieden substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im geradkettigen oder verzweigten Alkylteil steht, wobei als Cycloalkylsubstituenten jeweils infrage kommen: Halogen und/oder geradkettiges oder verzweigtes Alkyl mit 1 bis 3 Kohlenstoffatomen,
- R¹: außerdem für jeweils gegebenenfalls einfach bis fünffach, gleich oder verschieden substituiertes Aryl oder Arylalkyl mit jeweils 6 oder 10 Kohlenstoffatomen im Arylteil und gegebenenfalls 1 bis 4 Kohlenstoffatomen im geradkettigen oder verzweigten Alkylteil steht oder für jeweils gegebenenfalls einfach bis fünffach, gleich oder verschieden substituiertes, im Heterocyclylteil gesättigtes oder ungesättigtes Heterocyclyl oder Heterocyclylalkyl mit jweils 2 bis 9 Kohlenstoffatomen und 1 bis 3 gleichen oder verschiedenen Heteroatomen - insbesondere Stickstoff, Sauerstoff und/oder Schwefel - im Heterocyclylteil und gegebenenfalls 1 bis 4 Kohlenstoffatomen im geradkettigen oder verzweigten Alkylteil steht, wobei als Aryl- oder Heterocyclylsubstituenten jeweils die bei dem Substituenten R genannten infrage kommen,
- X: für Sauerstoff oder einen Rest der Formel N-R² steht,
- R²: für Wasserstoff oder für geradkettiges oder verzweigtes Alkyl mit 1 bis 4 Kohlenstoffatomen steht,
- m: für eine Zahl 0 oder 1 steht und
- n: für eine Zahl 0 oder 1 steht.

Ganz besonders bevorzugt sind Verbindungen der Formel (I), bei welchen
- R: für gegebenenfalls einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 2 Kohlenstoffatomen und 1 bis 5 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 3 Kohlenstoffatomen und 1 bis 5 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 3 Kohlenstoffatomen in den einzelnen Alkylteilen sowie jeweils gegebenenfalls einfach oder mehrfach, gleich oder verschieden durch Halogen und/oder jeweils geradkettiges oder verzweigtes Alkyl und/oder Alkoxy mit 1 bis 3 Kohlenstoffatomen substituiertes Phenyl, Phenoxy, Phenylthio, Phenylcarbonyl, Phenylcarbonyloxy, Phenylcarbonylamino, Phenylcarbonyl-(N-alkyl)-amino, Phenylaminocarbonyl, N-Phenyl-N-alkyl-aminocarbonyl, Phenylalkyl oder Phenylalkenyl mit jeweils gegebenenfalls bis zu 3 Kohlenstoffatomen in den einzelnen Alkyl- bzw. Alkenylteilen und
- R: außerdem für einen Rest der Formel 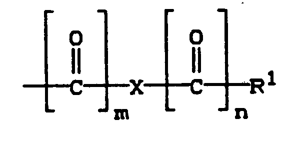 steht,
wobei
- R¹: für geradkettiges oder verzweigtes Alkyl mit 1 bis 3 Kohlenstoffatomen, für geradkettiges oder verzweigtes Halogenalkyl mit 1 bis 2 Kohlenstoffatomen und 1 bis 5 gleichen oder verschiedenen Halogenatomen, für jeweils geradkettiges oder verzweigtes Alkoxyalkyl oder Alkoxycarbonylalkyl mit jeweils 1 bis 3 Kohlenstoffatomen in den einzelnen Alkylteilen oder für jeweils gegebenenfalls einfach oder zweifach, gleich oder verschieden substituiertes Cyclopropyl, Cyclopentyl, Cyclohexyl, Cyclopropylalkyl, Cyclopentylalkyl oder Cyclohexylalkyl mit jeweils gegebenenfalls 1 bis 3 Kohlenstoffatomen im geradkettigen oder verzweigten Alkylteil steht, wobei als Cycloalkylsubstituenten jeweils infrage kommen: Fluor, Chlor, Brom und/oder geradkettiges oder verzweigtes Alkyl mit 1 bis 3 Kohlenstoffatomen,
- R¹: außerdem für jeweils gegebenenfalls einfach bis dreifach, gleich oder verschieden substituiertes Phenyl oder Phenylalkyl mit gegebenenfalls 1 bis 3 Kohlenstoffatomen im geradkettigen oder verzweigten Alkylteil steht oder für jeweils gegebenenfalls einfach bis dreifach, gleich oder verschieden substituiertes und/oder benzannelliertes Heterocyclyl oder Heterocyclylalkyl mit gegebenenfalls 1 bis 3 Kohlenstoffatomen im geradkettigen oder verzweigten Alkylteil steht, wobei als Heterocyclylreste insbesondere infrage kommen: Pyridinyl, Pyrimidinyl, Triazinyl, Imidazolyl, Triazolyl, Pyrazolyl, Thiazolyl, Isothiazolyl, Thiadiazolyl, Oxazolyl, Isoxazolyl und Oxadiazolyl, und wobei als Phenyl- oder Heterocyclylsubstituenten jeweils die bei dem Substituenten R genannten infrage kommen,
- X: für Sauerstoff oder einen Rest der Formel N-R² steht,
- R²: für Wasserstoff oder für geradkettiges oder verzweigtes Alkyl mit 1 bis 3 Kohlenstoffatomen steht,
- m: für eine Zahl 0 oder 1 steht und
- n: für eine Zahl 0 oder 1 steht.

Im einzelnen sei auf die bei den Herstellungsbeispielen genannten Verbindungen verwiesen.

Verwendet man beispielsweise 2-Acetamido-3-[3-(4-methylphenyl)-phenyl]-acrylsäure-N-methylamid und O-Methylhydroxylamin Hydrochlorid als Ausgangsstoffe, so läßt sich der Reaktionsablauf des erfindungsgemäßen Verfahrens (a) durch das folgende Formelschema darstellen: 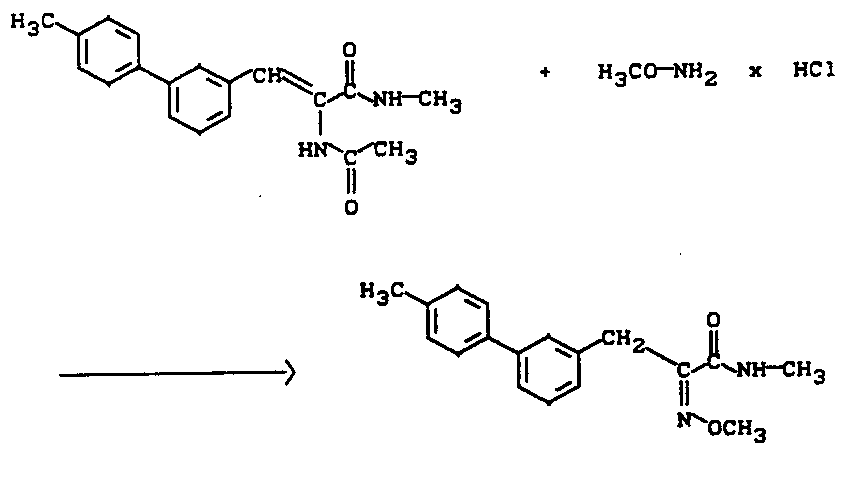
Verwendet man beispielsweise 3-(3-Hydroxyphenyl)-2-methoximino-propionsäure-N-methylamid und 4-Chlorbenzoylchlorid als Ausgangsstoffe, so läßt sich der Reaktionsablauf des erfindungsgemäßen Verfahrens (a) durch das folgende Formelschema darstellen: 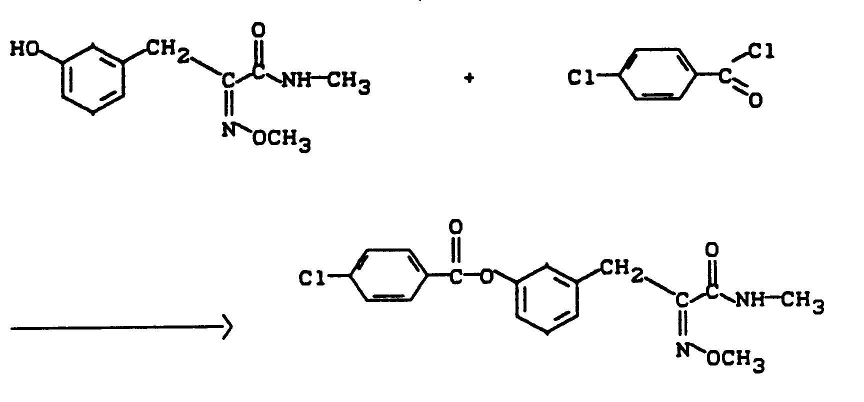
Die zur Durchführung des erfindungsgemäßen Verfahrens (a) als Ausgangsstoffe benötigten Acrylamid-Derivate sind durch die Formel (II) allgemein definiert. In dieser Formel (II) steht R vorzugsweise für diejenigen Reste, die bereits im Zusammenhang mit der Beschreibung der erfindungsgemäßen Stoffe der Formel (I) als bevorzugt für diese Substituenten genannt wurden. Die Acrylamid-Derivate der Formel (II))sind noch nicht bekannt und ebenfalls Gegenstand der Erfindung. Man erhält sie, wenn man aromatische Aldehyde der Formel (V), 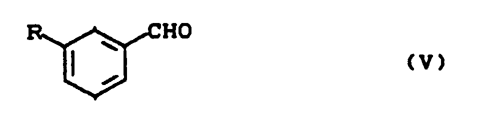
in welcher
- R: die oben angegebene Bedeutung hat,
mit N-Acetylglycin in Gegenwart von Acetanhydrid und Natriumacetat sowie gegebenenfalls in Gegenwart von Essigsaure bei Temperaturen zwischen 20°C und 160°C umsetzt und anschließend in einer zweiten Stufe die so erhältlichen Azlactone der Formel (VI), 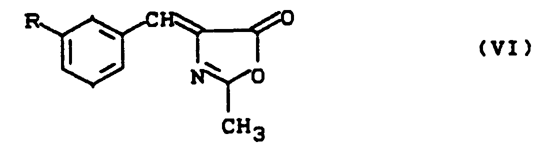
in welcher
- R: die oben angegebene Bedeutung hat,
mit Methylamin gegebenenfalls in Gegenwart eines Verdünnungsmittels bei Temperaturen zwischen 0°C und 50°C umsetzt.

Aromatische Aldehyde der Formel (V) sind allgemein bekannte Verbindungen der organischen Chemie oder erhältlich in Analogie zu allgemein bekannten Verfahren.

Azlactone der Formel (VI) sind bekannt (vergleiche z.B. DE 40 19 307).

Das zur Durchführung des erfindungsgemäßen Verfahrens (b) als Ausgangsverbindung benötigte 3-(3-Hydroxyphenyl)-2-methoximino-propionsäure-N-methylamid der Formel (III) ist noch nicht bekannt und ebenfalls Gegenstand der Erfindung.
Man erhält es, wenn man 3-Hydroxybenzaldehyd zunächst in einer ersten Stufe mit N-Acetylglycin in Gegenwart von Acetanhydrid und Natriumacetat sowie gegebenenfalls in Gegenwart von Essigsäure bei Temperaturen zwischen 20°C und 16°C umsetzt und anschließend in einer zweiten Stufe das so erhältliche Azlacton der Formel (VIa) 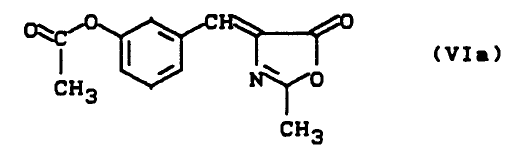
mit Methanol gegebenenfalls in Gegenwart einer Base, wie beispielsweise Natriumhydroxid und anschließend in Gegenwart einer Säure wie beispielsweise Salzsäure bei Temperaturen zwischen 0°C und 50°C umsetzt, danach in einer dritten Stufe das so erhältliche Acrylester-Derivat der Formel (VIIa) 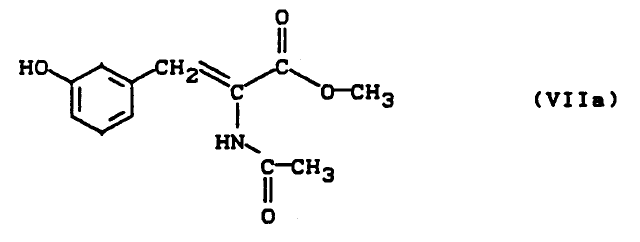
in Analogie zur Durchführung des erfindungsgemäßen Verfahrens (a) mit einem Säureadditionssalz des O-Methylhydroxylamins gegebenenfalls in Gegenwart eines Verdünnungsmittels und gegebenenfalls in Gegenwart eines Reaktionshilfsmittels umsetzt und schließlich den so erhältlichen Oximinoester der Formel (VIIIa) 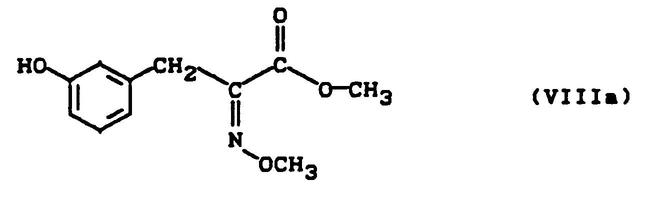
in einer vierten Stufe mit Methylamin gegebenenfalls in Gegenwart eines Verdünnungsmittels wie beispielsweise Tetrahydrofuran und gegebenenfalls in Gegenwart eines Reaktionshilfsmittels wie beispielsweise Eisessig bei Temperaturen zwischen 100°C und 150°C umsetzt.

Die zur Durchführung des erfindungsgemäßen Verfahrens (b) weiterhin als Ausgangsstoffe benötigten Alkylierungs- oder Acylierungsmittel sind durch die Formel (IV) allgemein definiert. In dieser Formel (IV) stehen R¹ und n vorzugsweise für diejenigen Reste und Indizes, die bereits im Zusammenhang mit der Beschreibung der erfindungsgemäßen Verbindungen der Formel (I) als bevorzugt für diesen Substituenten und Index genannt wurden.
E steht für eine bei Alkylierungsmitteln bzw. Acylierungsmitteln üblichen Abgangsrest, vorzugsweise für Halogen, insbesondere für Chlor, Brom oder Iod oder im Fall der Alkylierungsmittel für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy oder im Fall der Acylierungsmittel auch für einen Alkanoyloxyrest, wie insbesondere einen Acetoxy oder Propionyloxyrest.

Die Alkylierungs- und Acylierungsmittel der Formel (IV) sind allgemein bekannte Verbindungen der organischen Chemie.

Als Verdünnungsmittel zur Durchführung des erfindungsgemäßen Verfahrens (a) kommen polare organische Lösungsmittel oder wässrige Systeme infrage. Mit besonderem Vorzug verwendet man Alkohole, wie beispielsweise Methanol, Ethanol oder Propanol, deren Gemische mit Wasser oder reines Wasser als Verdünnungsmittel.

Als Reaktionshilfsmittel zur Durchführung des erfindungsgemäßen Verfahrens (a) kommen starke Mineralsäuren, wie Salzsäure, Bromwasserstoffsäure oder Schwefelsäure infrage.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 100°C, vorzugsweise bei Temperaturen zwischen 20°C und 60°C.

Zur Durchführung des erfindungsgemäßen Verfahrens (a) setzt man pro Mol an Acrylamid-Derivat der Formel (II) im allgemeinen 1.0 bis 3.0 Mol, vorzugsweise 1.0 bis 1.5 Mol an O-Methylhydroxylamin und gegebenenfalls 1.0 bis 3.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach bekannten Verfahren (vergleiche hierzu beispielsweise DE 40 19 307 oder die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkylbenzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6, oder Tris-[2-(2-methoxyethoxy)-ethyl]-amin.

Das erfindungsgemäße Verfahren (b) wird vorzugsweise in Gegenwart eines geeigneten Reaktionshilfsmittels durchgeführt. Als solche kommen alle üblichen anorganischen und organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-t-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und 100°C, vorzugsweise bei Temperaturen zwischen 0°C und 60°C.

Zur Durchführung des erfindungsgemäßen Verfahrens (b) setzt man pro Mol an 3-(3-Hydroxyphenyl)-2-methoximinopropionsäure-N-methylamid der Formel (III) im allgemeinen 1.0 bis 5.0 Mol, vorzugsweise 1.0 bis 1.5 Mol an Alkylierungs- oder Acylierungsmittel der Formel (IV) und gegebenenfalls 1.0 bis 5.0 Mol, vorzugsweise 1.0 bis 1.5 Mol an Reaktionshilfsmittel ein. Die Reaktionsdurchführung, Aufarbeitung und Isolierung der Reaktionsprodukte erfolgt nach bekannten Verfahren (vergleiche hierzu beispielsweise DE 40 19 307 oder die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten, wie beispielsweise gegen den Erreger des echten Getreidemehltaues an Gerste oder Weizen (Erysiphe graminis) oder gegen den Erreger der Netzfleckenkrankheit der Gerste (Pyrenophora teres) oder gegen den Erreger der Braunfleckenkrankheit an Gerste oder Weizen (Cochliobolus sativus) oder gegen den Erreger der Braunspelzigkeit des Weizens (Septoria nodorum) oder zur Bekämpfung von Krankheiten im Obst- und Gemüseanbau, wie beispielsweise gegen den Erreger des Apfelschorfes (Venturia inaequalis) oder zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) eingesetzt werden. Dabei zeigen die erfindungsgemäßen Wirkstoffe neben guten protektiven Eigenschaften auch systemische Wirksamkeit. Daneben besitzen die erfindungsgemäßen Wirkstoffe eine gute in vitro-Wirksamkeit.

Darüberhinaus eignen sich die Wirkstoffe auch zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..

Die erfindungsgemäßen Wirkstoffe zeichnen sich durch eine hohe insektizide Wirksamkeit aus.

Sie lassen sich mit besonders gutem Erfolg zur Bekämpfung von pflanzenschädigenden Insekten, wie beispielsweise gegen die Larven der Meerettichblattkäfer (Phaedon cochleariae) oder gegen die Raupen der Kohlschabe (Plutella Maculipennis) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffte, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung als Fungizide in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen dabei als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen bei der Verwendung als Fungizide im allgemeinen zwischen 1 und 0,0001 Gew.-%, vorzugsweise zwischen 0,5 und 0,001 %.

Bei der Saatgutbehandlung werden bei der Verwendung als Fungizide im allgemeinen Wirkstoffmengen von 0,001 bis 50 g je Kilogramm Saatgut, vorzugsweise 0,01 bis 10 g benötigt.

Bei Behandlung des Bodens sind bei der Verwendung als Fungizide Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können bei der Anwendung als Insektizide ebenfalls in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können bei der Anwendung als Insektizide ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann bei der Anwendung als Insektizide in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

### Herstellungsbeispiele:

### Beispiel 1

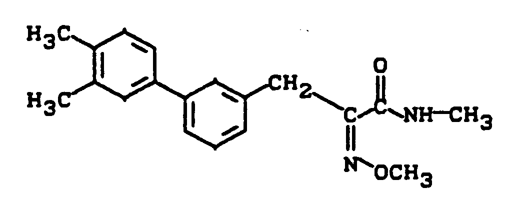

### (Verfahren a)

Zu 3,22 g (0,01 Mol) 2-Acetamido-3-[3-(3,4-dimethylphenyl)-phenyl]-acrylsäure-N-methylamid in 200 ml Methanol gibt man bei 60°C nacheinander 0,83 g (0,01 Mol) O-Methylhydroxylamin Hydrochlorid in 10 ml Wasser gelöst und 1 ml (0,01 Mol) konzentrierte Salzsäure und rührt anschließend 39 Stunden bei 60°C. Zur Aufarbeitung wird die Reaktionsmischung im Vakuum eingeengt, der Rückstand in 100 ml Dichlormethan aufgenommen, die organische Phase zweimal mit jeweils 50 ml Wasser gewaschen, über Natriumsulfat getrocknet und im Vakuum eingeengt. Der Rückstand wird über Kieselgel (Laufmittel: Dichlormethan) chromatographiert und anschließend aus n-Hexan kristallisiert.

Man erhält 1,5 g (48 % der Theorie) an 3-[3-(3,4-Dimethylphenyl)-phenyl]-2-methoximino-propionsäure-N-methylamid vom Schmelzpunkt 80°C.

### Herstellung der Ausgangsverbindung:

### Beispiel II-1

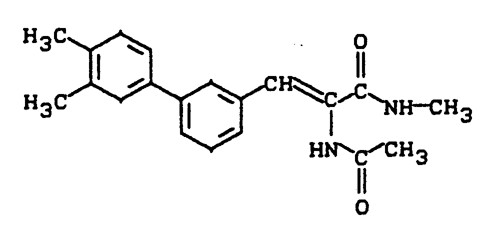

Zu 5,82 g (0,02 Mol) 2-Methyl-4-[3-(3,4-dimethylphenyl)-phenylmethyliden]-2,3-dehydro-1,3-oxazolidin-5-on in 100 ml Toluol gibt man bei Raumtemperatur 0,62 g (0,02 Mol) Methylamin in 2 ml Methanol gelöst zu und rührt anschließend 18 Stunden bei Raumtemperatur. Zur Aufarbeitung wird der ausgefallene Niederschlag abgesaugt und getrocknet.

Man erhält 5,6 g (87 % der Theorie) an 2-Acetamido-3-[3-(3,4-dimethylphenyl)-phenyl]-acrylsäure-N-methylamid vom Schmelzpunkt 224°C.

### Beispiel 2

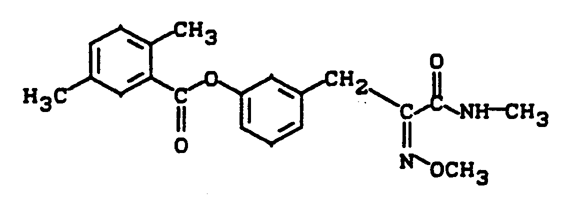

### (Verfahren b)

Zu 2,2 g (0,01 Mol) 3-(3-Hydroxyphenyl)-2-methoximinopropionsäure-N-methylamid in 50 ml Essigester gibt man 1,1 g (0,01 Mol) Triethylamin, anschließend tropfenweise unter Rühren bei Raumtemperatur 1,7 g (0,01 Mol) 2,5-Dimethylbenzoylchlorid und rührt nach beendeter Zugabe weitere 18 Stunden bei Raumtemperatur. Zur Aufarbeitung wird einmal mit 50 ml Wasser gewaschen, über Natriumsulfat getrocknet, im Vakuum eingeengt, der Rückstand mit Hilfe von Diisopropylether zur Kristallisation gebracht, abgesaugt und getrocknet.

Man erhält 2,6 g (73,4 % der Theorie) an 3-[3-(2,5-Dimethylbenzoyloxy)-phenyl]-2-methoximino-propionsäure-N-methylamid vom Schmelzpunkt 98°C.

### Herstellung der Ausgangsverbindung:

### Beispiel (III)

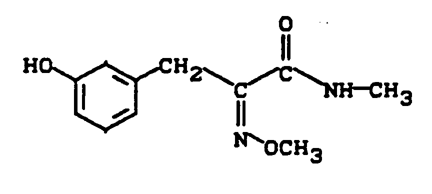

Zu 8,9 g (0,04 Mol) 3-(3-Hydroxyphenyl)-2-methoximino-propionsäure-methylester (vergleiche z. B. DE-OS 40 19 307) in 120 ml Tetrahydrofuran gibt man nacheinander 40 ml (0,4 Mol) 30prozentige wässrige Methylaminlösung und 4,8 g (0,08 Mol) Eisessig und erhitzt für 16 Stunden im Autoklaven auf 130°C. Zur Aufarbeitung wird das Lösungsmittel im Vakuum abdestilliert, der Rückstand in 150 ml Dichlormethan aufgenommen, die organische Phase zweimal mit jeweils 100 ml Wasser gewaschen, über Natriumsulfat getrocknet und im Vakuum eingeengt. Der Rückstand wird über Kieselgel (Laufmittel: Dichlormethan) chromatographiert und anschließend aus Diisopropylether umkristallisiert.

Man erhält 3,4 g (38,6 % der Theorie) an 3-(3-Hydroxyphenyl)-2-methoximino-propionsäure-N-methylamid vom Schmelzpunkt 100°C.

### Beispiel 3

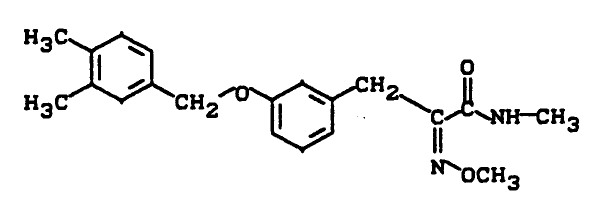

### (Verfahren b)

Zu 2,2 g (0,01 Mol) 3-(3-Hydroxyphenyl)-2-methoximino-propionsäure-N-methylamid in 100 ml Aceton gibt man nacheinander 1,4 g (0,01 Mol) gemahlenes Kaliumcarbonat, 2,0 g (0,01 Mol) 3,4-Dimethylbenzylbromid und eine Spatelspitze Kaliumiodid und rührt nach beendeter Zugabe 20 Stunden bei 60°C. Zur Aufarbeitung wird die Reaktionsmischung im Vakuum eingeengt, der Rückstand in 100 ml Dichlormethan aufgenommen, zweimal mit jeweils 50 ml Wasser gewaschen, über Natriumsulfat getrocknet, im Vakuum eingeengt, der Rückstand mit Hilfe von Diisopropylether zur Kristallisation gebracht, abgesaugt und getrocknet.

Man erhält 1,8 g (53 % der Theorie) an 3-[3-(3,4-Dimethylbenzyloxy)-phenyl]-2-methoximino-propionsäure-N-methylamid vom Schmelzpunkt 70°C.

In entsprechender Weise und gemäß den allgemeinen Angaben zur Herstellung erhält man die folgenden substituierten Oximetheramide der allgemeinen Formel (I):

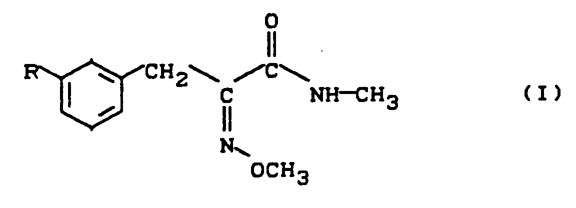

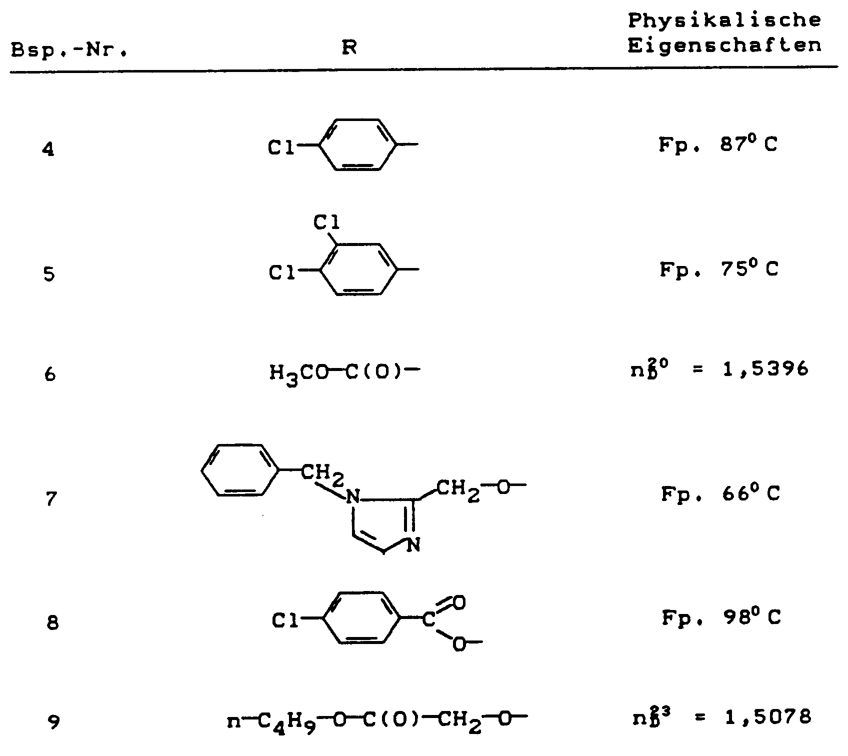

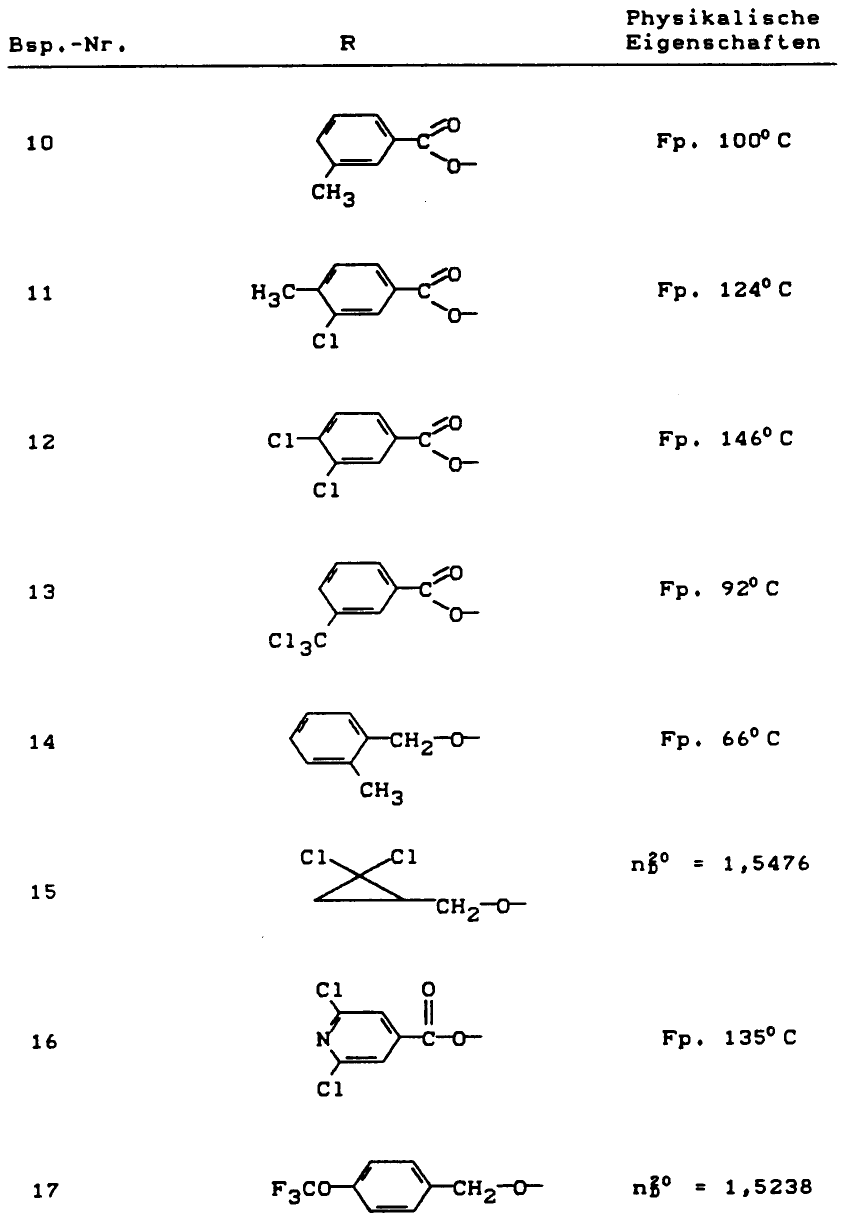

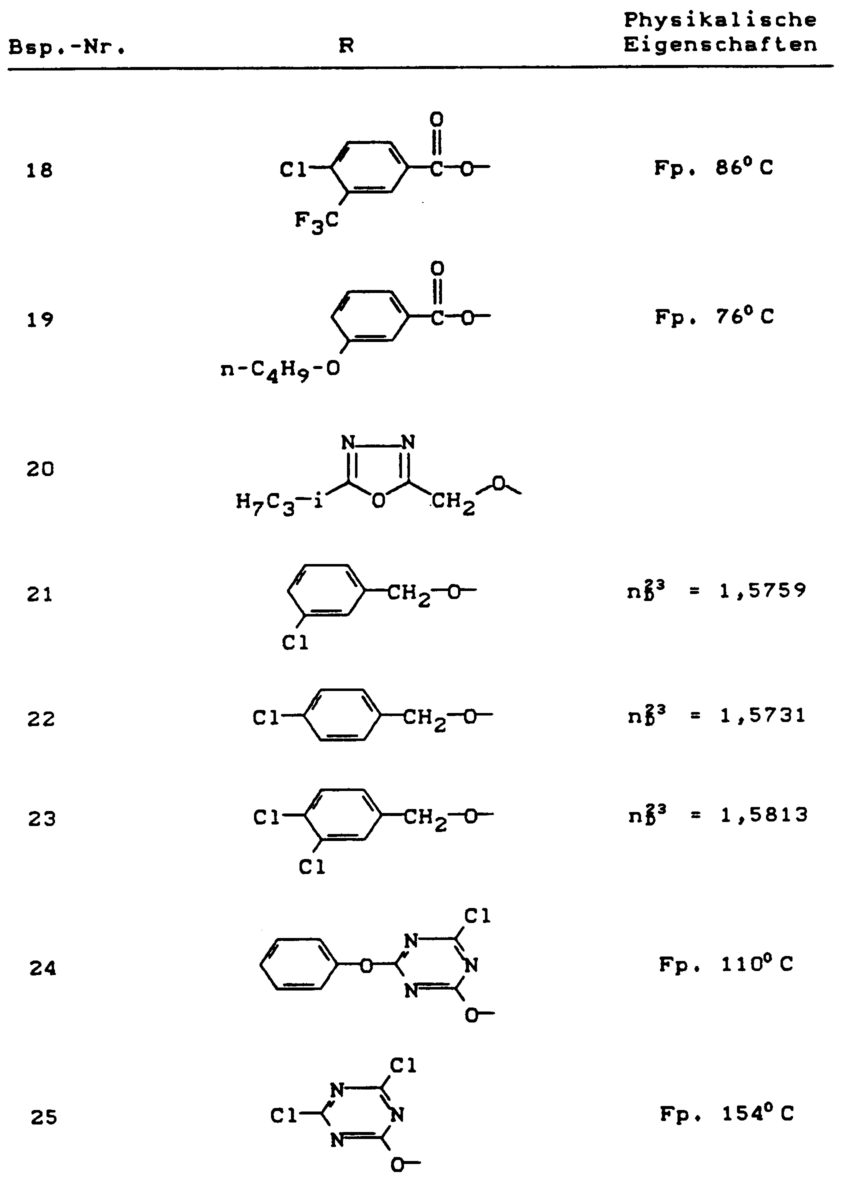

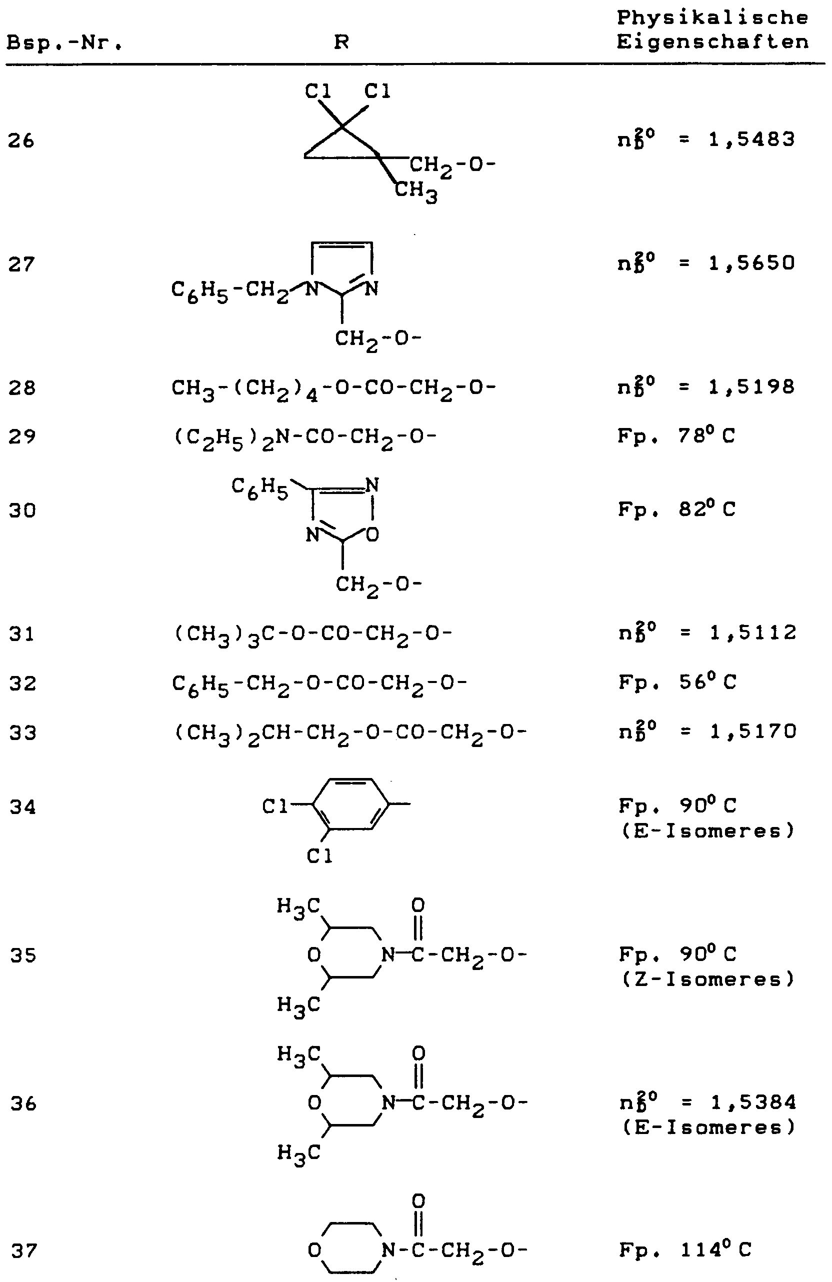

### Anwendungsbeispiele:

In den folgenden Anwendungsbeispielen wurden die nachstehend aufgeführten Verbindungen als Vergleichssubstanz eingesetzt: 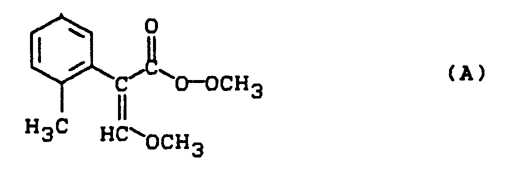
3-Methoxy-2-(2-methylphenyl)-acrylsäuremethylester 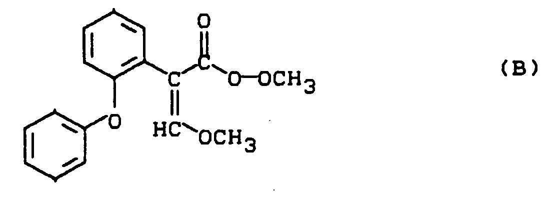
3-Methoxy-2-(2-phenoxyphenyl)-acrylsäuremethylester (beide bekannt aus EP 178 826)

### Beispiel A

### Venturia-Test (Apfel) / protektiv

Lösungsmittel: 4,7 Gewichtsteile Aceton
Emulgator: 0,3 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 2, 3, 4, 5, 13, 14, 16 und 18.

### Beispiel B

### Erysiphe-Test (Gerste) / protektiv

Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen folgender Herstellungsbeispiele 1 und 5, die bei einer Wirkstoffkonzentration von 250 ppm einen Wirkungsgrad von 100 % zeigen.

### Beispiel C

### Erysiphe-Test (Weizen) / protektiv

Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen Luftfeuchtigkeit von ca, 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 1.

### Beispiel D

### Erysiphe-Test (Gerste) / kurativ

Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 1.

### Beispiel E

### Erysiphe-Test (Weizen) / kurativ

Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. tritici bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 1.

### Beispiel F

### Phaedon Larven-Test

Lösungsmittel: 7 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerettichblattkäfer-Larven (Phaedon cochleariae) besetzt, solange die Blätter noch feucht sind.

Nach der gewünschten Zeit wird die Abtötung in Prozent bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegende Wirksamkeit gegenüber dem Stand der Technik: 4 und 5.

### Beispiel G

### Plutella-Test

Lösungsmittel: 7 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in Prozent bestimmt, Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegende Wirksamkeit gegenüber dem Stand der Technik: 4 und 5.

## Patentansprüche

1. Oximetheramide der Formel (I) 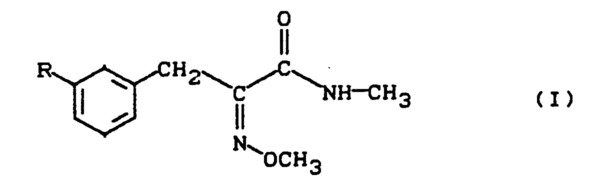 in welcher
R für gegebenenfalls substituiertes Aryl oder für einen Rest der Formel 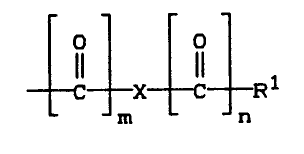 steht,
wobei
R¹ für Alkyl, Halogenalkyl, Alkoxyalkyl, Alkoxycarbonylalkyl oder für jeweils gegebenenfalls substituiertes Cycloalkyl, Cycloalkylalkyl, Aryl, Arylalkyl, Heterocyclyl oder Heterocyclylalkyl steht,
X für Sauerstoff oder einen Rest der Formel N-R² steht,
R² für Wasserstoff oder Alkyl steht,
m für eine Zahl 0 oder 1 steht und n für eine Zahl 0 oder 1 steht.
n für eine Zahl 0 oder 1 steht.

2. Verbindungen der Formel (I), gemäß Anspruch 1, bei welchen
R für gegebenenfalls einfach oder mehrfach, gleich oder verschieden substituiertes Aryl mit 6 bis 10 Kohlenstoffatomen steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 6 Kohlenstoffatomen, jeweils geradkettiges oder verzweigtes Halogenalkyl, Halogenalkoxy oder Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 6 Kohlenstoffatomen und 1 bis 11 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 6 Kohlenstoffatomen in den einzelnen Alkylteilen sowie jeweils gegebenenfalls einfach oder mehrfach, gleich oder verschieden durch Halogen und/oder geradkettiges oder verzweigtes Alkyl und/oder Alkoxy mit 1 bis 4 Kohlenstoffatomen substituiertes Phenyl, Phenoxy, Phenylthio, Phenylcarbonyl, Phenylcarbonyloxy, Phenylcarbonylamino, Phenylcarbonyl-(N-alkyl)-amino, Phenylaminocarbonyl, N-Phenyl-N-alkylaminocarbonyl, Phenylalkyl oder Phenylalkenyl mit jeweils gegebenenfalls bis zu 6 Kohlenstoffatomen in den einzelnen Alkyl- bzw. Alkenylteilen und
R außerdem für einen Rest der Formel 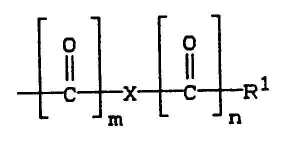 steht,
wobei
R¹ für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxyalkyl oder Alkoxycarbonylalkyl mit jeweils 1 bis 6 Kohlenstoffatomen in den einzelnen Alkylteilen oder für jeweils gegebenenfalls einfach oder mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil steht, wobei als Cycloalkylsubstituenten jeweils infrage kommen: Halogen und/oder geradkettiges oder verzweigtes Alkyl mit 1 bis 4 Kohlenstoffatomen,
R¹ außerdem für jeweils gegebenenfalls einfach oder mehrfach, gleich oder verschieden substituiertes Aryl oder Arylalkyl mit jeweils 6 bis 10 Kohlenstoffatomen im Arylteil und gegebenenfalls 1 bis 6 Kohlenstoffatomen im geradkettigen oder verzweigten Alkylteil steht oder für jeweils gegebenenfalls einfach oder mehrfach, gleich oder verschieden substituiertes, im Heterocyclylteil gesättigtes oder ungesättigtes Heterocyclyl oder Heterocyclylalkyl mit jeweils 2 bis 9 Kohlenstoffatomen und 1 bis 4 gleichen oder verschiedenen Heteroatomen - insbesondere Stickstoff, Sauerstoff und/oder Schwefel -im Heterocyclylteil und gegebenenfalls 1 bis 6 Kohlenstoffatomen im geradkettigen oder verzweigten Alkylteil steht, wobei als Aryl- oder Heterocyclylsubstituenten jeweils die bei dem Substituenten R genannten infrage kommen,
X für Sauerstoff oder einen Rest der Formel N-R² steht,
R² für Wasserstoff oder für geradkettiges oder verzweigtes Alkyl mit 1 bis 6 Kohlenstoffatomen steht,
m für eine Zahl 0 oder 1 steht und
n für eine Zahl 0 oder 1 steht.

3. Verbindungen der Formel (I), gemäß Anspruch 1, bei welchen
R für gegebenenfalls einfach bis fünffach, gleich oder verschieden substituiertes Aryl mit 6 oder 10 Kohlenstoffatomen steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 5 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 5 Kohlenstoffatomen und 1 bis 9 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 4 Kohlenstoffatomen in den einzelnen Alkylteilen sowie jeweils gegebenenfalls einfach oder mehrfach, gleich oder verschieden durch Halogen und/oder jeweils geradkettiges oder verzweigtes Alkyl und/oder Alkoxy mit jeweils 1 bis 3 Kohlenstoffatomen substituiertes Phenyl, Phenoxy, Phenylthio, Phenylcarbonyl, Phenylcarbonyloxy, Phenylcarbonylamino, Phenylcarbonyl-(N-alkyl)-amino, Phenylaminocarbonyl, N-Phenyl-N-alkyl-aminocarbonyl, Phenylalkyl oder Phenylalkenyl mit jeweils gegebenenfalls bis zu 4 Kohlenstoffatomen in den einzelnen Alkyl- bzw. Alkenylteilen und
R außerdem für einen Rest der Formel 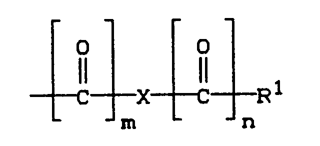 steht,
wobei
R¹ für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkoxycarbonylalkyl mit jeweils 1 bis 4 Kohlenstoffatomen in den einzelnen Alkylteilen oder für jeweils gegebenenfalls einfach bis vierfach, gleich oder verschieden substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im geradkettigen oder verzweigten Alkylteil steht, wobei als Cycloalkylsubstituenten jeweils infrage kommen: Halogen und/oder geradkettiges oder verzweigtes Alkyl mit 1 bis 3 Kohlenstoffatomen,
R¹ außerdem für jeweils gegebenenfalls einfach bis fünffach, gleich oder verschieden substituiertes Aryl oder Arylalkyl mit jeweils 6 oder 10 Kohlenstoffatomen im Arylteil und gegebenenfalls 1 bis 4 Kohlenstoffatomen im geradkettigen oder verzweigten Alkylteil steht oder für jeweils gegebenenfalls einfach bis fünffach, gleich oder verschieden substituiertes, im Heterocyclylteil gesättigtes oder ungesättigtes Heterocyclyl oder Heterocyclylalkyl mit jweils 2 bis 9 Kohlenstoffatomen und 1 bis 3 gleichen oder verschiedenen Heteroatomen - insbesondere Stickstoff, Sauerstoff und/oder Schwefel - im Heterocyclylteil und gegebenenfalls 1 bis 4 Kohlenstoffatomen im geradkettigen oder verzweigten Alkylteil steht, wobei als Aryl- oder Heterocyclylsubstituenten jeweils die bei dem Substituenten R genannten infrage kommen,
X für Sauerstoff oder einen Rest der Formel N-R² steht,
R² für Wasserstoff oder für geradkettiges oder verzweigtes Alkyl mit 1 bis 4 Kohlenstoffatomen steht,
m für eine Zahl 0 oder 1 steht und
n für eine Zahl 0 oder 1 steht.

4. Verbindungen der Formel (I), gemäß Anspruch 1, bei welchen
R für gegebenenfalls einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Alkenyl, Alkinyl, Alkenyloxy, Alkinyloxy, Alkenylthio oder Alkinylthio mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 2 Kohlenstoffatomen und 1 bis 5 gleichen oder verschiedenen Halogenatomen, jeweils geradkettiges oder verzweigtes Halogenalkenyl, Halogenalkinyl, Halogenalkenyloxy, Halogenalkinyloxy, Halogenalkenylthio oder Halogenalkinylthio mit jeweils 2 bis 3 Kohlenstoffatomen und 1 bis 5 gleichen oder verschiedenen Halogenatomen, jeweils geradkettiges oder verzweigtes Alkylcarbonyl, Alkoxycarbonyl, Alkylcarbonyloxy, Alkylcarbonylamino, Alkylcarbonyl-(N-alkyl)-amino, Aminocarbonyl, N-Alkyl-aminocarbonyl, N,N-Dialkylaminocarbonyl oder Alkoximinoalkyl mit jeweils 1 bis 3 Kohlenstoffatomen in den einzelnen Alkylteilen sowie jeweils gegebenenfalls einfach oder mehrfach, gleich oder verschieden durch Halogen und/oder jeweils geradkettiges oder verzweigtes Alkyl und/oder Alkoxy mit 1 bis 3 Kohlenstoffatomen substituiertes Phenyl, Phenoxy, Phenylthio, Phenylcarbonyl, Phenylcarbonyloxy, Phenylcarbonylamino, Phenylcarbonyl-(N-alkyl)-amino, Phenylaminocarbonyl, N-Phenyl-N-alkyl-aminocarbonyl, Phenylalkyl oder Phenylalkenyl mit jeweils gegebenenfalls bis zu 3 Kohlenstoffatomen in den einzelnen Alkyl- bzw. Alkenylteilen und
R außerdem für einen Rest der Formel 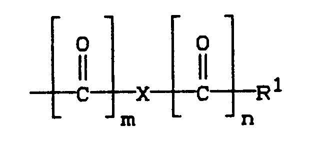 steht,
wobei
R¹ für geradkettiges oder verzweigtes Alkyl mit 1 bis 3 Kohlenstoffatomen, für geradkettiges oder verzweigtes Halogenalkyl mit 1 bis 2 Kohlenstoffatomen und 1 bis 5 gleichen oder verschiedenen Halogenatomen, für jeweils geradkettiges oder verzweigtes Alkoxyalkyl oder Alkoxycarbonylalkyl mit jeweils 1 bis 3 Kohlenstoffatomen in den einzelnen Alkylteilen oder für jeweils gegebenenfalls einfach oder zweifach, gleich oder verschieden substituiertes Cyclopropyl, Cyclopentyl, Cyclohexyl, Cyclopropylalkyl, Cyclopentylalkyl oder Cyclohexylalkyl mit jeweils gegebenenfalls 1 bis 3 Kohlenstoffatomen im geradkettigen oder verzweigten Alkylteil steht, wobei als Cycloalkylsubstituenten jeweils infrage kommen: Fluor, Chlor, Brom und/oder geradkettiges oder verzweigtes Alkyl mit 1 bis 3 Kohlenstoffatomen,
R¹ außerdem für jeweils gegebenenfalls einfach bis dreifach, gleich oder verschieden substituiertes Phenyl oder Phenylalkyl mit gegebenenfalls 1 bis 3 Kohlenstoffatomen im geradkettigen oder verzweigten Alkylteil steht oder für jeweils gegebenenfalls einfach bis dreifach, gleich oder verschieden substituiertes und/oder benzannelliertes Heterocyclyl oder Heterocyclylalkyl mit gegebenenfalls 1 bis 3 Kohlenstoffatomen im geradkettigen oder verzweigten Alkylteil steht, wobei als Heterocyclylreste insbesondere infrage kommen: Pyridinyl, Pyrimidinyl, Triazinyl, Imidazolyl, Triazolyl, Pyrazolyl, Thiazolyl, Isothiazolyl, Thiadiazolyl, Oxazolyl, Isoxazolyl und Oxadiazolyl, und wobei als Phenyl- oder Heterocyclylsubstituenten jeweils die bei dem Substituenten R genannten infrage kommen,
X für Sauerstoff oder einen Rest der Formel N-R² steht,
R² für Wasserstoff oder für geradkettiges oder verzweigtes Alkyl mit 1 bis 3 Kohlenstoffatomen steht,
m für eine Zahl 0 oder 1 steht und
n für eine Zahl 0 oder 1 steht.

5. Schädlingsbekämpfungsmittel, gekennzeichnet durch einen Gehalt an mindestens einen Oxiimetheramid der Formel (I) nach Anspruch 1.

6. Verfahren zur Bekämpfung von Schädlingen, dadurch gekennzeichnet, daß man Oximetheramide der Formel (I) nach Anspruch 1 auf Schädlinge und/oder ihren Lebensraum einwirken läßt.

7. Verfahren zur Herstellung von substituierten Oximetheramiden der allgemeinen Formel (I), 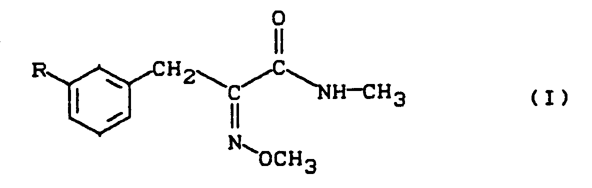 in welcher
R für gegebenenfalls substituiertes Aryl oder für einen Rest der Formel 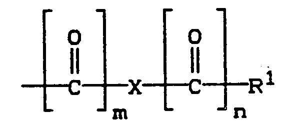 steht,
wobei
R¹ für Alkyl, Halogenalkyl, Alkoxyalkyl, Alkoxycarbonylalkyl oder für jeweils gegebenenfalls substituiertes Cycloalkyl, Cycloalkylalkyl, Aryl, Arylalkyl, Heterocyclyl oder Heterocyclylalkyl steht,
X für Sauerstoff oder einen Rest der Formel N-R² steht,
R² für Wasserstoff oder Alkyl steht,
m für eine Zahl 0 oder 1 steht und
n für eine Zahl 0 oder 1 steht,
dadurch gekennzeichnet, daß man
a) Acrylamid-Derivate der Formel (II), 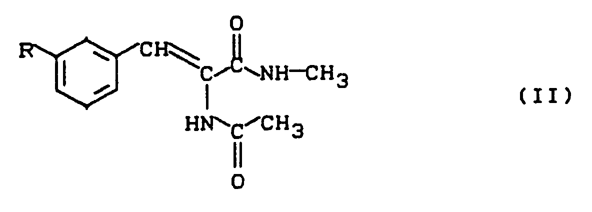 in welcher
R die oben angegebene Bedeutung hat,
mit einem Säureadditionssalz des O-Methylhydroxylamins gegebenenfalls in Gegenwart eines Verdünnungsmittels und gegebenenfalls in Gegenwart eines Reaktionshilfsmittels umsetzt oder wenn man
b) 3-(3-Hydroxyphenyl)-2-methoximino-propionsäure-N-methylamid der Formel (III), 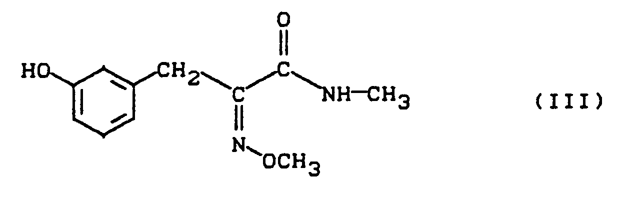 mit Alkylierungs- oder Acylierungsmitteln der Formel (IV), 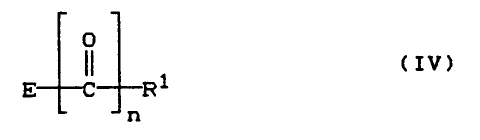 in welcher
R¹ und n die oben angegebene Bedeutung haben und
E für eine elektronenanziehende Abgangsgruppe steht,
gegebenenfalls in Gegenwart eines Verdünnungsmittels und gegebenenfalls in Gegenwart eines Reaktionshilfsmittels acyliert oder alkyliert.

8. Verwendung von Oximetheramiden der Formel (I) nach den Ansprüchen 1 bis 4 zur Bekämpfung von Schädlingen.

9. Verfahren zur Herstellung von Schädlingsbekämpfungsmitteln, dadurch gekennzeichnet, daß man Oximetheramide der Formel (I) nach den Ansprüchen 1 bis 4 mit Streckmitteln und/oder Oberflächenaktiven Mitteln vermischt.

10. Acrylamid-Derivate der Formel (II) 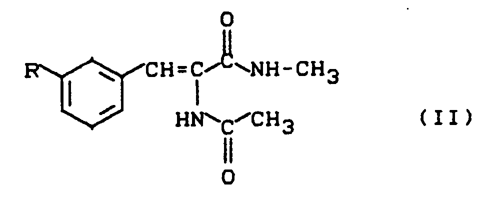 in welcher R die in Anspruch 1 angegebene Bedeutung hat.

11. 3-(3-Hydroxyphenyl)-2-methoximinopropionsäure-N-methylamid der Formel (III)

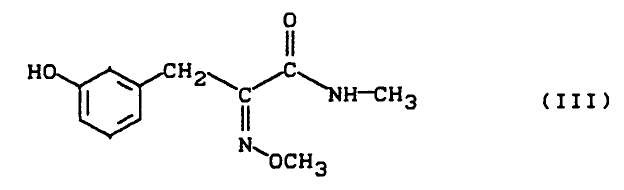

## Claims

1. Oxime ether amides of the formula (I) 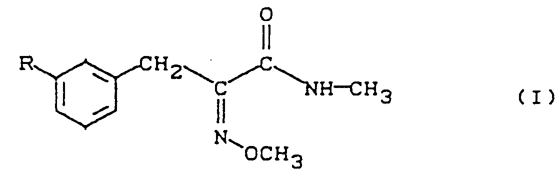 in which
R represents optionally substituted aryl or a radical of the formula 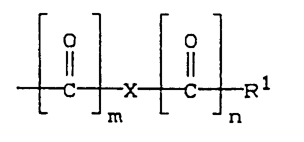 where
R¹ represents alkyl, halogenoalkyl, alkoxyalkyl or alkoxycarbonylalkyl, or represents in each case optionally substituted cycloalkyl, cycloalkylalkyl, aryl, arylalkyl, heterocyclyl or heterocyclylalkyl,
X represents oxygen or a radical of the formula N-R²,
R² represents hydrogen or alkyl,
m represents a number 0 or 1 and
n represents a number 0 or 1.

2. Compounds of the formula (I) according to Claim 1, in which
R represents aryl which has 6 to 10 carbon atoms and which is optionally monosubstituted or polysubstituted by identical or different substituents, suitable substituents being:
halogen, cyano, nitro, in each case straight-chain or branched alkyl, alkoxy, alkylthio, alkylsulphinyl or alkylsulphonyl, each of which has 1 to 6 carbon atoms, in each case straight-chain or branched alkenyl, alkinyl, alkenyloxy, alkinyloxy, alkenylthio or alkinylthio, each of which has 2 to 6 carbon atoms, in each case straight-chain or branched halogenoalkyl, halogenoalkoxy or halogenoalkylthio, halogenoalkylsulphinyl or halogenoalkylsulphonyl, each of which has 1 to 6 carbon atoms and 1 to 13 identical or different halogen atoms, in each case straight-chain or branched halogeno-alkenyl, halogenoalkinyl, halogenoalkenyloxy, halogenoalkinyloxy, halogenoalkenylthio or halogenoalkinylthio, each of which has 2 to 6 carbon atoms and 1 to 11 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl, each of which has 1 to 6 carbon atoms in the individual alkyl moieties, and also phenyl, phenoxy, phenylthio, phenylcarbonyl, phenylcarbonyloxy, phenylcarbonylamino, phenylcarbonyl-(N-alkyl)-amino, phenylaminocarbonyl, N-phenyl-N-alkyl-aminocarbonyl, phenylalkyl or phenylalkenyl, each of which has, where appropriate, up to 6 carbon atoms in the individual alkyl or alkenyl moieties and each of which is optionally monosubstituted or polysubstituted by identical or different substituents from the series comprising halogen and/or straight-chain or branched alkyl and/or alkoxy having 1 to 4 carbon atoms, and
R furthermore represents a radical of the formula 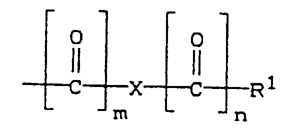 where
R¹ represents straight-chain or branched alkyl having 1 to 6 carbon atoms, straight-chain or branched halogenoalkyl having 1 to 6 carbon atoms and 1 to 13 identical or different halogen atoms, in each case straight-chain or branched alkoxyalkyl or alkoxycarbonylalkyl, each of which has 1 to 6 carbon atoms in the individual alkyl moieties, or represents cycloalkyl or cycloalkylalkyl, each of which has 3 to 7 carbon atoms in the cycloalkyl moiety and, if appropriate, 1 to 6 carbon atoms in the straight-chain or branched alkyl moiety and each of which is optionally monosubstituted or polysubstituted by identical or different substituents, suitable cycloalkyl substituents in each case being: halogen and/or straight-chain or branched alkyl having 1 to 4 carbon atoms,
R¹ furthermore represents aryl or arylalkyl, each of which has 6 to 10 carbon atoms in the aryl moiety and, if appropriate, 1 to 6 carbon atoms in the straight-chain or branched alkyl moiety and each of which is optionally monosubstituted or polysubstituted by identical or different substituents, or represents heterocyclyl or heterocyclylalkyl, each of which has 2 to 9 carbon atoms and 1 to 4 identical or different hetero atoms - in particular nitrogen, oxygen and/or sulphur - in the heterocyclyl moiety and, if appropriate, 1 to 6 carbon atoms in the straight-chain or branched alkyl moiety, and each of which is optionally monosubstituted or polysubstituted in the heterocyclyl moiety by identical or different substituents, suitable aryl substituents or heterocyclyl substituents in each case being those mentioned in the case of the substituent R,
X represents oxygen or a radical of the formula N-R²,
R² represents hydrogen or straight-chain or branched alkyl having 1 to 6 carbon atoms,
m represents a number 0 or 1 and
n represents a number 0 or 1.

3. Compounds of the formula (1) according to Claim 1, in which
R represents aryl which has 6 to 10 carbon atoms and which is optionally monosubstituted to pentasubstituted by identical or different substituents, suitable substituents being: halogen, cyano, nitro, in each case straight-chain or branched alkyl, alkoxy, alkylthio, alkylsulphinyl or alkylsulphonyl, each of which has 1 to 4 carbon atoms, in each case straight-chain or branched alkenyl, alkinyl, alkenyloxy, alkinyloxy, alkenylthio or alkinylthio, each of which has 2 to 5 carbon atoms, in each case straight-chain or branched halogenoalkyl, halogenoalkoxy or halogenoalkylthio, halogenoalkylsulphinyl or halogenoalkylsulphonyl, each of which has 1 to 4 carbon atoms and 1 to 9 identical or different halogen atoms, in each case straight-chain or branched halogenoalkenyl, halogenoalkinyl, halogenoalkenyloxy, halogenoalkinyloxy, halogenoalkenylthio or halogenoalkinylthio, each of which has 2 to 5 carbon atoms and 1 to 9 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl, each of which has 1 to 4 carbon atoms in the individual alkyl moieties, and also phenyl, phenoxy, phenylthio, phenylcarbonyl, phenylcarbonyloxy, phenylcarbonylamino, phenylcarbonyl-(N-alkyl)-amino, phenylaminocarbonyl, N-phenyl-N-alkyl-aminocarbonyl, phenylalkyl or phenylalkenyl, each of which has, where appropriate, up to 4 carbon atoms in the individual alkyl or alkenyl moieties and each of which is optionally monosubstituted or polysubstituted by identical or different substituents from the series comprising halogen and/or in each case straight-chain or branched alkyl and/or alkoxy, each of which has 1 to 3 carbon atoms, and
R furthermore represents a radical of the formula 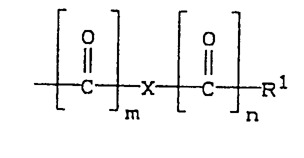 where
R¹ represents straight-chain or branched alkyl having 1 to 4 carbon atoms, or represents straight-chain or branched halogenoalkyl having 1 to 4 carbon atoms and 1 to 9 identical or different halogen atoms, or represents in each case straight-chain or branched alkoxyalkyl or alkoxycarbonylalkyl, each of which has 1 to 4 carbon atoms in the individual alkyl moieties, or represents cycloalkyl or cycloalkylalkyl, each of which has 3 to 6 carbon atoms in the cycloalkyl moiety and, if appropriate, 1 to 4 carbon atoms in the straight-chain or branched alkyl moiety and each of which is optionally monosubstituted to tetrasubstituted by identical or different substituents, suitable cycloalkyl substituents in each case being: halogen and/or straight-chain or branched alkyl having 1 to 3 carbon atoms,
R¹ furthermore represents aryl or arylalkyl, each of which has 6 or 10 carbon atoms in the aryl moiety and, if appropriate, 1 to 4 carbon atoms in the straight-chain or branched alkyl moiety and each of which is optionally monosubstituted to pentasubstituted by identical or different substituents, or represents heterocyclyl or heterocyclylalkyl, each of which has 2 to 9 carbon atoms and 1 to 3 identical or different hetero atoms - in particular nitrogen, oxygen and/or sulphur - in the heterocyclyl moiety and, if appropriate, 1 to 4 carbon atoms in the straight-chain or branched alkyl moiety, and each of which is optionally saturated or unsaturated in the heterocyclyl moiety and each of which is optionally monosubstituted to pentasubstituted by identical or different substituents, suitable aryl substituents or heterocyclyl substituents being those mentioned in the case of the substituent R,
X represents oxygen or a radical of the formula N-R²,
R² represents hydrogen or straight-chain or branched alkyl having 1 to 4 carbon atoms,
m represents a number 0 or 1 and
n represents a number 0 or 1.

4. Compounds of the formula (I) according to Claim 1, in which
R represents phenyl which is optionally monosubstituted to trisubstituted by identical or different substituents, suitable substituents being:
halogen, cyano, nitro, in each case straightchain or branched alkyl, alkoxy, alkylthio, alkylsulphinyl or alkylsulphonyl, each of which has 1 to 3 carbon atoms, in each case straight-chain or branched alkenyl, alkinyl, alkenyloxy, alkinyloxy, alkenylthio or alkinylthio, each of which has 2 to 4 carbon atoms, in each case straight-chain or branched halogenoalkyl, halogenoalkoxy, halogenoalkylthio, halogenoalkylsulphinyl or halogenoalkylsulphonyl, each of which has 1 to 2 carbon atoms and 1 to 5 identical or different halogen atoms, in each case straight-chain or branched halogenoalkenyl, halogeno-alkinyl, halogenoalkenyloxy, halogenoalkinyloxy, halogenoalkenylthio or halogeno-alkinylthio, each of which has 2 to 3 carbon atoms and 1 to 5 identical or different halogen atoms, in each case straight-chain or branched alkylcarbonyl, alkoxycarbonyl, alkylcarbonyloxy, alkylcarbonylamino, alkylcarbonyl-(N-alkyl)-amino, aminocarbonyl, N-alkyl-aminocarbonyl, N,N-dialkylaminocarbonyl or alkoximinoalkyl, each of which has 1 to 3 carbon atoms in the individual alkyl moieties, and also phenyl, phenoxy, phenylthio, phenylcarbonyl, phenylcarbonyloxy, phenylcarbonylamino, phenylcarbonyl-(N-alkyl)-amino, phenylaminocarbonyl, N-phenyl-N-alkyl-aminocarbonyl, phenylalkyl or phenylalkenyl, each of which has, where appropriate, up to 3 carbon atoms in the individual alkyl or alkenyl moieties and each of which is optionally monosubstituted or polysubstituted by identical or different substituents from the series comprising halogen and/or in each case straight-chain or branched alkyl and/or alkoxy, having 1 to 3 carbon atoms, and
R furthermore represents a radical of the formula 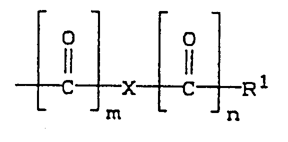 where
R¹ represents straight-chain or branched alkyl having 1 to 3 carbon atoms, or represents straight-chain or branched halogenoalkyl having 1 to 2 carbon atoms and 1 to 5 identical or different halogen atoms, in each case straight-chain or branched alkoxyalkyl or alkoxycarbonylalkyl, each of which has 1 to 3 carbon atoms in the individual alkyl moieties, or represents cyclopropyl, cyclopentyl, cyclohexyl, cyclopropylalkyl, cyclo-pentylalkyl or cyclohexylalkyl, each of which has, where appropriate, 1 to 3 carbon atoms in the straight-chain or branched alkyl moiety and each of which is optionally monosubstituted or disubstituted by identical or different substituents, suitable cycloalkyl substituents in each case being: fluorine, chlorine, bromine and/or straight-chain or branched alkyl having 1 to 3 carbon atoms,
R¹ furthermore represents phenyl or phenylalkyl which has, where appropriate, 1 to 3 carbon atoms in the straight-chain or branched alkyl moiety and each of which is optionally monosubstituted to trisubstituted by identical or different substituents, or represents heterocyclyl or heterocyclylalkyl which has, if appropriate, 1 to 3 carbon atoms in the straight-chain or branched alkyl moiety and each of which is optionally monosubstituted to trisubstituted by identical or different substituents and/or benzo-fused, particularly suitable heterocyclyl radicals being: pyridinyl, pyrimidinyl, triazinyl, imidazolyl, triazolyl, pyrazolyl, thiazolyl, isothiazolyl, thiadiazolyl, oxazolyl, isoxazolyl and oxadiazolyl, and suitable phenyl or heterocyclyl substitutents in each case being those mentioned in the case of the substituent R,
X represents oxygen or a radical of the formula N-R²,
R² represents hydrogen or straight-chain or branched alkyl having 1 to 3 carbon atoms,
m represents a number 0 or 1 and
n represents a number 0 or 1.

5. Pesticides, characterised in that they contain at least one oxime ether amide of the formula (I) according to Claim 1.

6. Method of combating pests, characterised in that oxime ether amides of the formula (I) according to Claim 1 are allowed to act on pests and/or their environment.

7. Process for the preparation of substituted oxime ether amides of the general formula (I) 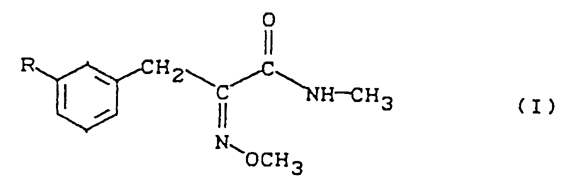 in which
R represents optionally substituted aryl or a radical of the formula 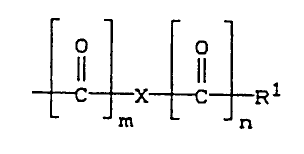 where
R¹ represents alkyl, halogenoalkyl, alkoxyalkyl or alkoxycarbonylalkyl, or represents in each case optionally substituted cycloalkyl, cycloalkylalkyl, aryl, arylalkyl, heterocyclyl or heterocyclylalkyl,
X represents oxygen or a radical of the formula N-R²,
R² represents hydrogen or alkyl,
m represents a number 0 or 1 and
n represents a number 0 or 1,
characterized in that
a) acrylamide derivatives of the formula (II) 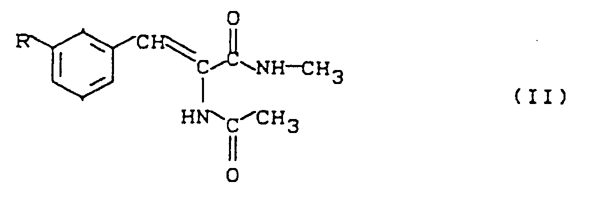 in which
R has the abovementioned meaning
are reacted with an acid addition salt of O-methylhydroxylamine, if appropriate in the presence of a diluent and if appropriate in the presence of a reaction auxiliary, or when
b) N-methyl-3-(3-hydroxyphenyl)-2-methoximino-propionamide, of the formula (III), 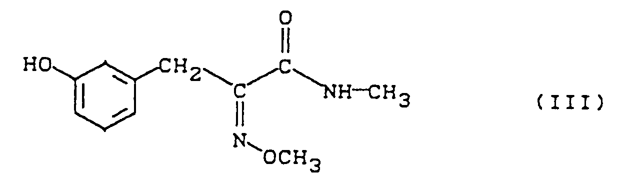 is acylated or alkylated with alkylating or acylating agents of the formula (IV) 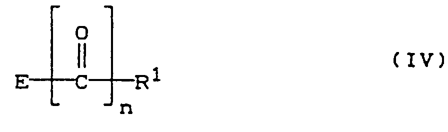 in which
R¹ and n have the abovementioned meaning and
E represents an electron-attracting leaving group,
if appropriate in the presence of a diluent and if appropriate in the presence of a reaction auxiliary.

8. Use of oxime ether amides of the formula (I) according to Claims 1 to 4 for combating pests.

9. Process for the preparation of pesticides, characterised in that oxime ether amides of the formula (I) according to Claims 1 to 4 are mixed with extenders and/or surface-active agents.

10. Acrylamide derivatives of the formula (II) 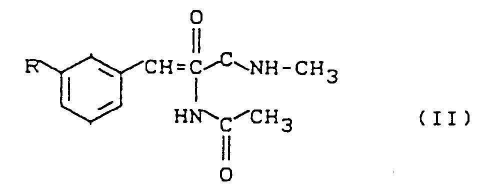 in which R has the meaning given in Claim 1.

11. N-Methyl-3-(3-hydroxyphenyl)-2-methoximinopropionamide, of the formula (III)

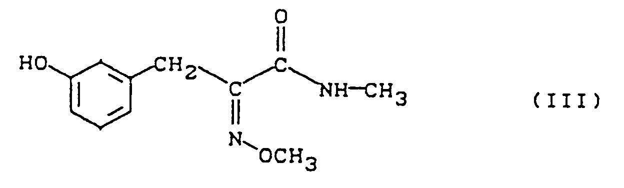

## Revendications

1. Oximétheramides de formule (I) 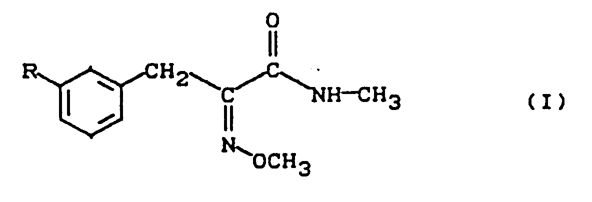 dans laquelle
R est un groupe aryle éventuellement substitué ou un reste de formule 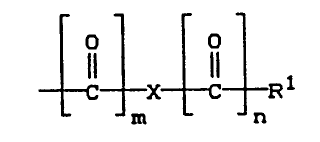 dans laquelle
R¹ est un groupe alkyle, halogénalkyle, alkoxyalkyle, alkoxycarbonylalkyle ou un groupe cycloalkyle, cycloalkylalkyle, aryle, arylalkyle, hétérocyclyle ou hétérocyclylalkyle dont chacun est éventuellement substitué,
X est de l'oxygène ou un reste de formule N-R²,
R² est de l'hydrogène ou un groupe alkyle,
m est le nombre 0 ou 1 et
n est le nombre 0 ou 1.

2. Composés de formule (I) suivant la revendication 1, dans lesquels
R est un groupe aryle de 6 à 10 atomes de carbone portant, le cas échéant, un ou plusieurs substituants identiques ou différents, les substituants considérés étant les suivants :
un halogène, un groupe cyano, nitro, un groupe alkyle, alkoxy, alkylthio, alkylsulfinyle ou alkylsulfonyle linéaire ou ramifié ayant chacun 1 à 6 atomes de carbone, un groupe alcényle, alcynyle, alcényloxy, alcynyloxy, alcénylthio ou alcynylthio linéaire ou ramifié ayant chacun 2 à 6 atomes de carbone, un groupe halogénalkyle, halogénalkoxy, halogénalkylthio, halogénalkylsulfinyle ou halogénalkylsulfonyle linéaire ou ramifié ayant chacun 1 à 6 atomes de carbone et 1 à 13 atomes d'halogènes identiques ou différents, un groupe halogénalcényle, halogénalcynyle, halogénalcényloxy, halogénalcynyloxy, halogénalcénylthio ou halogénalcynylthio linéaire ou ramifié ayant chacun 2 à 6 atomes de carbone et 1 à 11 atomes d'halogènes identiques ou différents, un groupe alkylcarbonyle, alkoxycarbonyle, alkylcarbonyloxy, alkylcarbonylamino, alkylcarbonyl-(N-alkyl)-amino, aminocarbonyle, N-alkylaminocarbonyle, N-N-dialkylaminocarbonyle ou alkoximinoalkyle linéaire ou ramifié ayant chacun 1 à 6 atomes de carbone dans les parties alkyle individuelles ainsi qu'un groupe phényle, phénoxy, phénylthio, phénylcarbonyle, phénylcarbonyloxy, phénylcarbonylamino, phénylcarbonyl-(N-alkyl)-amino, phénylaminocarbonyle, N-phényl-N-alkylaminocarbonyle, phénylalkyle ou phénylalcényle ayant chacun jusqu'à 6 atomes de carbone dans les parties alkyle ou alcényle individuelles, portant, le cas échéant, un ou plusieurs substituants halogéno et/ou alkyle et/ou alkoxy linéaires ou ramifiés en C₁ à C₄ identiques ou différents, et
R représente en outre un reste de formule 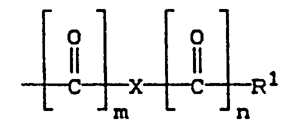 dans laquelle
R¹ est un groupe alkyle linéaire ou ramifié ayant 1 à 6 atomes de carbone, un groupe halogénalkyle linéaire ou ramifié ayant 1 à 6 atomes de carbone et 1 à 13 atomes d'halogènes identiques ou différents, un groupe alkoxyalkyle ou alkoxycarbonylalkyle linéaire ou ramifié ayant chacun 1 à 6 atomes de carbone dans les parties alkyle individuelles ou un groupe cycloalkyle ou cycloalkylalkyle ayant chacun 3 à 7 atomes de carbone dans la partie cycloalkyle et, le cas échéant, 1 à 6 atomes de carbone dans la partie alkyle linéaire ou ramifiée, portant, le cas échéant, un ou plusieurs substituants identiques ou différents, en considérant dans chaque cas comme substituants de la partie cycloalkyle un halogène et/ou un groupe alkyle linéaire ou ramifié ayant 1 à 4 atomes de carbone,
R¹ représente en outre un groupe aryle ou arylalkyle ayant chacun 6 à 10 atomes de carbone dans la partie aryle et, le cas échéant, 1 à 6 atomes de carbone dans la partie alkyle linéaire ou ramifiée, portant chacun, le cas échéant, un ou plusieurs substituants identiques ou différents, ou un groupe hétérocyclyle ou hétérocyclylalkyle saturé ou non saturé dans la partie hétérocyclyle, ayant chacun 2 à 9 atomes de carbone et 1 à 4 hétéro-atomes - notamment azote, oxygène et/ou soufre - identiques ou différents dans la partie hétérocyclyle et, le cas échéant, 1 à 6 atomes de carbone dans la partie alkyle linéaire ou ramifiée, portant chacun, le cas échéant, un ou plusieurs substituants identiques ou différents, les substituants considérés pour les parties aryle ou hétérocyclyle étant dans chaque cas ceux qui ont été mentionnés pour le substituant R,
X est de l'oxygène ou un reste de formule N-R²,
R² est de l'hydrogène ou un groupe alkyle linéaire ou ramifié ayant 1 à 6 atomes de carbone,
m a la valeur 0 ou 1 et
n a la valeur 0 ou 1.

3. Composés de formule (I) suivant la revendication 1, dans lesquels
R est un groupe aryle ayant 6 ou 10 atomes de carbone portant, le cas échéant, un à cinq substituants identiques ou différents, les substituants considérés étant les suivants :
halogène, groupe cyano, nitro, groupe alkyle, alkoxy, alkylthio, alkylsulfinyle ou alkylsulfonyle linéaire ou ramifié ayant chacun 1 à 4 atomes de carbone, groupe alcényle, alcynyle, alcényloxy, alcynyloxy, alcénylthio ou alcynylthio linéaire ou ramifié ayant chacun 2 à 5 atomes de carbone, groupe halogénalkyle, halogénalkoxy, halogénalkylthio, halogénalkylsulfinyle ou halogénalkylsulfonyle linéaire ou ramifié ayant chacun 1 à 4 atomes de carbone et 1 à 9 atomes d'halogènes identiques ou différents, groupe halogénalcényle, halogénalcynyle, halogénalcényloxy, halogénalcynyloxy, halogénalcénylthio ou halogénalcynylthio linéaire ou ramifié ayant chacun 2 à 5 atomes de carbone et 1 à 9 atomes d'halogènes identiques ou différents, groupe alkylcarbonyle, alkoxycarbonyle, alkylcarbonyloxy, alkylcarbonylamino, alkylcarbonyl-(N-alkyl)-amino, aminocarbonyle, N-alkylaminocarbonyle, N,N-dialkylaminocarbonyle ou alkoximinoalkyle linéaire ou ramifié ayant chacun 1 à 4 atomes de carbone dans les parties alkyle individuelles, ainsi qu'un groupe phényle, phénoxy, phénylthio, phénylcarbonyle, phénylcarbonyloxy, phénylcarbonylamino, phénylcarbonyl-(N-alkyl)-amino, phénylaminocarbonyle, N-phényl-N-alkylaminocarbonyle, phénylalkyle ou phénylalcényle ayant chacun, le cas échéant, jusqu'à 4 atomes de carbone dans les parties alkyle ou alcényle individuelles et portant chacun, le cas échéant, un ou plusieurs substituants halogéno et/ou alkyle et/ou alkoxy linéaires ou ramifiés en C₁ à C₃ identiques ou différents et
R représente en outre un reste de formule 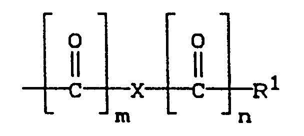 dans laquelle
R¹ est un groupe alkyle linéaire ou ramifié ayant 1 à 4 atomes de carbone, un groupe halogénalkyle linéaire ou ramifié ayant 1 à 4 atomes de carbone et 1 à 9 atomes d'halogènes identiques ou différents, un groupe alkoxyalkyle ou alkoxycarbonylalkyle linéaire ou ramifié ayant chacun 1 à 4 atomes de carbone dans les parties alkyle individuelles ou un groupe cycloalkyle ou cycloalkylalkyle ayant chacun 3 à 6 atomes de carbone dans la partie cycloalkyle et, le cas échéant, 1 à 4 atomes de carbone dans la partie alkyle linéaire ou ramifiée, portant chacun, le cas échéant, 1 à 4 substituants identiques ou différents, en considérant dans chaque cas comme substituants de la partie cycloalkyle : un halogène et/ou un groupe alkyle linéaire ou ramifié ayant 1 à 3 atomes de carbone,
R¹ représente en outre un groupe aryle ou arylalkyle ayant chacun 6 ou 10 atomes de carbone dans la partie aryle et, le cas échéant, 1 à 4 atomes de carbone dans la partie alkyle linéaire ou ramifiée, portant chacun, le cas échéant, un à cinq substituants identiques ou différents, ou un groupe hétérocyclyle ou hétérocyclylalkyle saturé ou non saturé dans la partie hétérocyclyle, ayant chacun 2 à 9 atomes de carbone et 1 à 3 hétéro-atomes - notamment azote, oxygène et/ou soufre - identiques ou différents dans la partie hétérocyclyle et, le cas échéant, 1 à 4 atomes de carbone dans la partie alkyle linéaire ou ramifiée, portant chacun, le cas échéant, un à cinq substituants identiques ou différents, en considérant dans chaque cas comme substituants des parties aryle ou hétérocyclyle ceux qui sont mentionnés dans le cas du substituant R,
X est de l'oxygène ou un reste de formule N-R²,
R² est de l'hydrogène ou un reste alkyle linéaire ou ramifié ayant 1 à 4 atomes de carbone,
m est le nombre 0 ou 1 et
n est le nombre 0 ou 1.

4. Composés de formule (I) suivant la revendication 1, dans lesquels
R est un groupe phényle portant, le cas échéant, un à trois substituants identiques ou différents, les substituants considérés étant les suivants :
halogène, groupe cyano, nitro, groupe alkyle, alkoxy, alkylthio, alkylsulfinyle ou alkylsulfonyle linéaire ou ramifié ayant chacun 1 à 3 atomes de carbone, groupe alcényle, alcynyle, alcényloxy, alcynyloxy, alcénylthio ou alcynylthio linéaire ou ramifié ayant chacun 2 à 4 atomes de carbone, groupe halogénalkyle, halogénalkoxy, halogénalkylthio, halogénalkylsulfinyle ou halogénalkylsulfonyle linéaire ou ramifié ayant chacun 1 ou 2 atomes de carbone et 1 à 5 atomes d'halogènes identiques ou différents, groupe halogénalcényle, halogénalcynyle, halogénalcényloxy, halogénalcynyloxy, halogénalcénylthio ou halogénalcynylthio linéaire ou ramifié ayant chacun 2 ou 3 atomes de carbone et 1 à 5 atomes d'halogènes identiques ou différents, groupe alkylcarbonyle, alkoxycarbonyle, alkylcarbonyloxy, alkylcarbonylamino, alkylcarbonyl-(N-alkyl)-amino, aminocarbonyle, N-alkylaminocarbonyle, N,N-dialkylaminocarbonyle ou alkoximinoalkyle linéaire ou ramifié ayant chacun 1 à 3 atomes de carbone dans les parties alkyle individuelles, ainsi que groupe phényle, phénoxy, phénylthio, phénylcarbonyle, phénylcarbonyloxy, phénylcarbonylamino, phénylcarbonyl-(N-alkyl)-amino, phénylaminocarbonyle, N-phényl-N-alkylaminocarbonyle, phénylalkyle ou phénylalcényle ayant chacun, le cas échéant, jusqu'à 3 atomes de carbone dans les parties alkyle ou alcényle individuelles, portant chacun, le cas échéant, un ou plusieurs substituants identiques ou différents, halogéno et/ou alkyle et/ou alkoxy linéaires ou ramifiés ayant 1 à 3 atomes de carbone et
R représente en outre un reste de formule 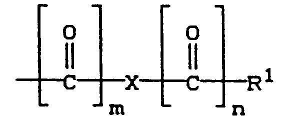 dans laquelle
R¹ est un groupe alkyle linéaire ou ramifié ayant 1 à 3 atomes de carbone, un groupe halogénalkyle linéaire ou ramifié ayant 1 ou 2 atomes de carbone et 1 à 5 atomes d'halogènes identiques ou différents, un groupe alkoxyalkyle ou alkoxycarbonylalkyle linéaire ou ramifié ayant chacun 1 à 3 atomes de carbone dans les parties alkyle individuelles ou un groupe cyclopropyle, cyclopentyle, cyclohexyle, cyclopropylalkyle, cyclopentylalkyle ou cyclohexylalkyle ayant chacun, le cas échéant, 1 à 3 atomes de carbone dans la partie alkyle linéaire ou ramifiée et portant chacun, le cas échéant, un ou deux substituants identiques ou différents, en considérant dans chaque cas comme substituants du groupe cycloalkyle : le fluor, le chlore, le brome et/ou un radical alkyle linéaire ou ramifié ayant 1 à 3 atomes de carbone,
R¹ représente en outre un groupe phényle ou phénylalkyle ayant, le cas échéant, 1 à 3 atomes de carbone dans la partie alkyle linéaire ou ramifiée, portant chacun, le cas échéant, un à trois substituants identiques ou différents, ou un groupe hétérocyclyle ou hétérocyclylalkyle ayant, le cas échéant, 1 à 3 atomes de carbone dans la partie alkyle linéaire ou ramifiée, portant chacun, le cas échéant, un à trois substituants identiques ou différents, et/ou étant condensés au benzène, les restes hétérocyclyle considérés étant en particulier les suivants : pyridinyle, pyrimidinyle, triazinyle, imidazolyle, triazolyle, pyrazolyle, thiazolyle, isothiazolyle, thiadiazolyle, oxazolyle, isoxazolyle ou oxadiazolyle, et les substituants des restes phényle ou hétérocyclyle étant dans chaque cas ceux qui ont été mentionnés pour le substituant R,
X est de l'oxygène ou un reste de formule N-R²,
R² est de l'hydrogène ou un reste alkyle linéaire ou ramifié ayant 1 à 3 atomes de carbone,
m a la valeur 0 ou 1 et
n a la valeur 0 ou 1.

5. Compositions pesticides, caractérisées par une teneur en au moins un oximétheramide de formule (I) suivant la revendication 1.

6. Procédé pour combattre des parasites, caractérisé en ce qu'on fait agir des oximétheramides de formule (I) suivant la revendication 1 sur les parasites et/ou sur leur milieu.

7. Procédé de production d'oximétheramides substitués de formule générale (I), 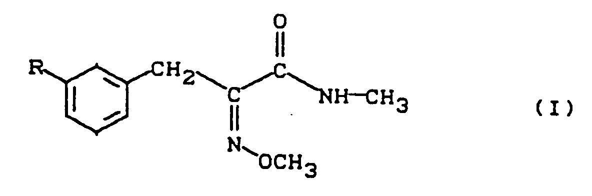 dans laquelle
R est un reste aryle éventuellement substitué ou un reste de formule 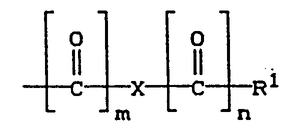 dans laquelle
R¹ est un groupe alkyle, halogénalkyle, alkoxyalkyle, alkoxycarbonylalkyle ou un reste cycloalkyle, cycloalkylalkyle, aryle, arylalkyle, hétérocyclyle ou hétérocyclylalkyle dont chacun est éventuellement substitué,
X est de l'oxygène ou un reste de formule N-R²,
R² est de l'hydrogène ou un groupe alkyle,
m a la valeur 0 ou 1 et
n a la valeur 0 ou 1,
caractérisé en ce que
a) on fait réagir des dérivés d'acrylamide de formule (II), 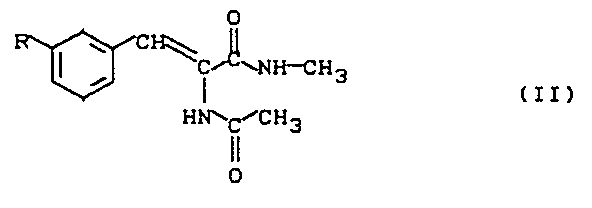 dans laquelle
R a la définition indiquée ci-dessus,
avec un sel d'addition d'acide de la O-méthylhydroxylamine, le cas échéant en présence d'un diluant et en la présence éventuelle d'une substance auxiliaire de réaction, ou bien
b) on acyle ou alkyle le N-méthylamide de l'acide 3-(3-hydroxyphényl)-2-méthoximinopropionique de formule (III), 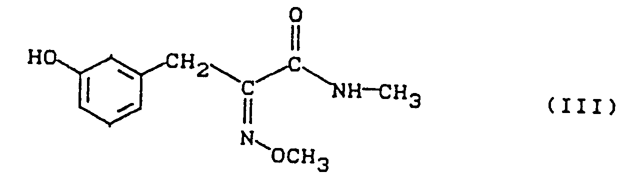 avec des agents d'alkylation ou d'acylation de formule (IV), 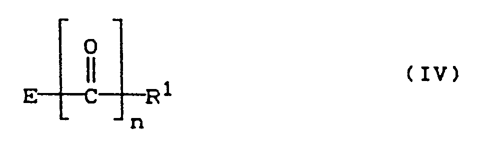 dans laquelle
R¹ et n ont la définition indiquée ci-dessus et
E est un groupe partant attirant les électrons,
le cas échéant, en présence d'un diluant et en la présence éventuelle d'une substance auxiliaire de réaction.

8. Utilisation d'oximétheramides de formule (I) suivant les revendications 1 à 4 pour combattre des parasites.

9. Procédé de préparation de compositions pesticides, caractérisé en ce qu'on mélange des oximétheramides de formule (I) suivant les revendications 1 à 4 avec des diluants et/ou des agents tensio-actifs.

10. Dérivés d'acrylamide de formule (II) 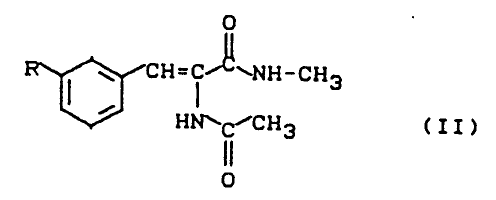 dans laquelle R a la définition indiquée dans la revendication 1.

11. N-méthylamide d'acide 3-(3-hydroxyphényl)-2-méthoximinopropionique de formule (III)
